# EUROPEAN PATENT SPECIFICATION

(11) **EP 2 391 351 B1**
(45) Date of publication and mention of the grant of the patent: **20.08.2014**
(21) Application number: 10701246.0
(22) Date of filing: 27.01.2010
(51) Int. Cl.: A61K 9/20, A61K 31/4015

(54) **Pharmaceutical compositions comprising 2-oxo-1-pyrrolidine derivatives**
Pharmazeutische Zusammensetzungen, die 2-oxo-1-Pyrrolidin-derivate umfassen
Compositions pharmaceutiques comprenant des dérivés de 2-oxo-1-pyrrolidine

(30) Priority: 29.01.2009 EP 09100084
(43) Date of publication of application: 07.12.2011
(73) Proprietor: UCB Pharma, S.A., 1070 Brussels (BE)
(72) Inventor: CUYPERS, Serge, B-1070 Brussels (BE); BERWAER, Monique, B-1070 Brussels (BE); FANARA, Domenico, B-1070 Brussels (BE); BARILLARO, Valery, B-1070 Brussels (BE); LARBANOIX, Martine, B-1070 Brussels (BE); DARGELAS, Frédéric, B-1050 Brussels (BE)
(74) Representative: UCB Intellectual Property
(86) International application number: PCT/EP2010/050893
(87) International publication number: WO 2010/086315

(56) References cited:
- WO-A-2006/131322
- U.S. National Institutes of Health: "Clinical Study in Healthy Volunteers to Investigate the Neurocognitive Effects of a New Antiepileptic Drug: Brivaracetam" ClinicalTrialsFeeds.org 27 October 2008 (2008-10-27), XP002572305 Retrieved from the Internet: URL:http://clinicaltrialsfeeds.org/clinica l-trials/show/NCT00736931>
- SHANGRAW R F ET AL: "Characterization of the tableting properties of [beta]-cyclodextrin and the effects of processing variables on inclusion complex formation, compactibility and dissolution" DRUG DEVELOPMENT AND INDUSTRIAL PHARMACY, NEW YORK, NY, US, vol. 18, no. 17, 1 November 1992 (1992-11-01), pages 1831-1851, XP008106571 ISSN: 0363-9045
- "Cyclodextrins" In: ROWE R C ET AL: "Handbook of pharmaceutical excipients. Fifth edition", 2006, PHARMACEUTICAL PRESS, London, UK pages 217-221,

## Description

The present invention relates to a pharmaceutical composition of 2-oxo-1-pyrrolidine derivatives, a process of the preparation thereof and therapeutic uses thereof.

International patent application having publication number WO 01/62726 discloses 2-oxo-1-pyrrolidine derivatives and methods for their preparation. It particularly discloses compound (2S)-2-[(4R)-2-oxo-4-propyl-pyrrolidin-1-yl] butanamide known under the international non propriety name of Brivaracetam.

International patent application having publication number WO 2005/121082 describes a process of preparation of 2-oxo-1-pyrrolidine derivatives and particularly discloses a process of preparation of (2S)-2-[(4S)-4-(2,2-difluorovinyl)-2-oxo-pyrrolidin-1-yl]butanamide known under the international non propriety name of Seletracetam.

2-oxo-1-pyrrolidine derivatives are therefore particularly useful in the pharmaceutical industry.

Brivaracetam is effective in the treatment of epilepsy. U.S. National Institutes of Health: "Clinical Study in Healthy Volunteers to Investigate the Neurocognitive Effects of a New Antiepileptic Drug: Brivaracetam", ClinicalTrialsFeeds.org, 27 October 2008 (http://clinicaltrialsfeeds.org/clinical-trials/show/NCT00736931 discloses tablets comprising the antiepileptic drug brivaracetam. A clinical trial evaluated the efficacy and safety of Brivaracetam (5, 20 and 50 mg per day) in the adjunctive treatment of adult patients with refractory partial onset seizures, with or without secondary generalization. Brivaracetam is also effective in the treatment of patients with post-herpetic neuralgia.

Seletracetam is effective in the treatment of epilepsy. Two studies were conducted with Seletracetam in epilepsy evaluating the efficacy and safety of Seletracetam in the adjunctive treatment of partial onset seizures in highly refractory adult patients currently receiving up to three concomitant anti-epileptic drugs.

One of the objectives of the invention is a pharmaceutical composition which can be administered orally to obtain an immediate release of pharmaceutically active substances.

Considering Brivaracetam and Seletracetam are classified as BCS I, the resulting *in vitro* dissolution (USP <711> apparatus n°2) according to the Guidance for Industry Immediate Release Solid Oral Dosage Forms the composition, In Vitro Dissolution Testing, (Center for Drug Evaluation and Research November 1995) should meet the criterion of the test described in the Case A of the Dissolution Documentation: Dissolution of 85% in 15 minutes in 900 mL of 0.1 N HCl. If it fails it should meet the test described in Case B or C.

As a general rule the term of Immediate Release is understood here as not being a modified or controlled released and having an in-vitro dissolution release (USP <711> apparatus n°2 of a least 75% in 45 min in an appropriate buffered aqueous media.

The present invention relates to an oral pharmaceutical composition, in a solid tablet form, comprising an active ingredient and 0.1 % to 60% per weight of at least a cyclodextrin agent, with respect to the total weight of the composition, the active ingredient being an 2-oxo-1-pyrrolidine derivative of formula (I), 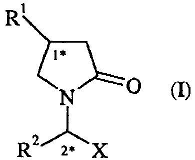 wherein,
R¹ is C₁₋₁₀ alkyl or C₂₋₆ alkenyl;
R² is C₁₋₁₀ alkyl or C₂₋₆ alkenyl;
X is -CONR⁴R⁵, -COOH, -COOR³ or -CN;
R³ is C₁₋₁₀ alkyl;
R⁴ is hydrogen or C₁₋₁₀ alkyl;
R⁵ is hydrogen or C₁₋₁₀ alkyl.

The term "active ingredient" as used herein is defined as a substance or a drug which has a therapeutic effect. It can also be a mixture of substances having a therapeutic effect.

The amount of the active ingredient present in the pharmaceutical composition of the invention may vary depending on the patient to which the compositions are administered and the disease to be treated.

The oral composition of the invention is in a solid form. The oral composition is in a form of a homogeneous blend. The oral composition includes only one phase without taking into account the coating, in fact the composition is free of particules and of granules. The oral composition is in a form of a homogeneous blend of the active ingredient and excipients which are not agglomerated.

The oral composition of the invention is a tablet.

The term "alkyl", as used herein, is a group which represents saturated, monovalent hydrocarbon radicals having straight (unbranched), branched or cyclic moieties, or combinations thereof. Preferred alkyl comprises 1 to 10 carbons. More preferred alkyl comprises 1 to 4 carbons. Optionally, alkyl groups may be substituted by 1 to 5 substituents independently selected from the group consisting of halogen, hydroxy, alkoxy, ester, acyl, cyano, acyloxy, acid, amide or amino group. Preferred alkyl groups are methyl, ethyl, n-propyl, trifluoromethyl and trifluoroethyl.

The term "alkenyl" as used herein represents unsubstituted or substituted branched, unbranched or cyclic hydrocarbon radicals or combinations thereof having at least one double bond. Preferred alkenyl comprises 2 to 6 carbons. More preferred alkenyl comprises 2 to 4 carbons. "Alkenyl" moieties may be optionally substituted by 1 to 5 substituents independently selected from the group consisting of halogen, hydroxy, alkoxy, ester, acyl, cyano, acyloxy, carboxylic acid, amide or amino group.

The term "halogen", as used herein, represents an atom of fluorine, chlorine, bromine, or iodine.

The term "hydroxy", as used herein, represents a group of formula -OH.

The term "alkoxy", as used herein, represents a group of formula -OR^{a} wherein R^{a} is C₁₋₄ alkyl as defined above.

The term "acyl" as used herein, represents a group of formula R^{b}CO-, wherein R^{b} represents a C₁₋₄ alkyl as defined above.

The term "ester", as used herein, represents a group of formula -COOR^{c} wherein R^{c} represents a C₁₋₄ alkyl as defined above.

The term "cyano" as used herein represents a group of formula -CN.

The term "acyloxy" as used herein represents a group of formula -O-COR^{d}, wherein R^{d} is a C₁₋₄ alkyl as defined above or an aryl group.

The term "aryl" as used herein, represents an organic radical derived from an aromatic hydrocarbon by removal of one hydrogen, for example a phenyl.

The term "carboxylic acid" as used herein represents a group of formula-COOH.

The term "amino group", as used herein, represents a group of formula -NH₂, NHR^{e} or NR^{f}R^{e} wherein R^{e} and R^{f} are alkyl groups as defined above in the specification.

The term "amide", as used herein, refers to a group of formula -CO-NH₂, -CO-NHR^{g}, or -CO-NR^{g}R^{h}, wherein R^{g} and R^{h} are alkyl groups as defined above in the specification.

The term "sulfonate group" as used herein represents a group of formula -0-SO₂-Rⁱ wherein Rⁱ is an alkyl or an aryl as defined here above in the specification. Preferred sulfonate groups are methanesulfonate, *para*-toluenesulfonate group or trifluoromethanesulfonate.

In one embodiment, according to first aspect of the present invention, R¹ is C₁₄ alkyl or C₂₋₄ alkenyl. In a further embodiment according to first aspect of the present invention, R¹ is n-propyl or 2,2-difluorovinyl.

In one embodiment according to first aspect of the present invention, R² is C₁₋₄ alkyl. In another embodiment according to first aspect of the present invention, R² is ethyl.

In one embodiment according to first aspect of the present invention, X is-CONR⁴R⁵, -COOH or -COOR³, wherein R³ is a C₁₋₄ alkyl. In another embodiment according to first aspect of the present invention, X is -CONR⁴R⁵.

In a particular embodiment, R³ is methyl.

In one embodiment according to first aspect of the present invention, R⁴ is hydrogen or C₁₋₄ alkyl. In another embodiment according to first aspect of the present invention, R⁴ is hydrogen.

In one embodiment according to first aspect of the present invention, R⁵ is hydrogen or C₁₋₄ alkyl. In another embodiment according to the first aspect of the present invention, R⁵ is hydrogen.

Preferably R¹ is n-propyl or 2,2-difluorovinyl; R² is ethyl; and X is -CONH₂.

More preferably, the active ingredient is chosen among brivaracetam and seletracetam. Best results have been obtained with brivaracetam.

The term "cyclodextrin agent" as used herein is defined as a pharmaceutical acceptable excipient which is a cyclic oligosaccharide created by 6, 7 or 8 alpha-D-glucopyrannose units, commonly known as alpha, beta or gamma cyclodextrin respectively. It is added as a binding agent. Usually, the cyclodextrin agent is chosen among alpha cyclodextrin, beta cyclodextrin, hydroxypropyl beta cyclodextrin, methyl beta cyclodextrin, sulfobutyl beta cyclodextrin, gamma cyclodextrin, and hydroxypropyl gamma cyclodextrin. Generally, the cyclodextrin agent is a beta cyclodextrin. Preferably, the cyclodextrin agent is a beta cyclodextrin having a crystalline structure by contrast to amorphous cyclodextrin. In a preferred embodiment, the cyclodextrin agent is a beta cyclodextrin having a water content between 4 and 18 %, and preferably between 5 and 16 % (w/w), and more preferably between 10 and 16 % (w/w). The best results have been obtained with a beta cyclodextrin having a water content between 10 and 14 % (w/w).

More preferably, the cyclodextrin agent is the beta cyclodextrin sold under the trademark Kleptose® or Betadex ®, or Cavamax® W7.

Usually, the pharmaceutical composition according to the present invention comprises 0.1 to 50 % per weight of cyclodextrin agent with respect to the total weight of the composition. Particularly, the pharmaceutical composition comprises 0.1 to 45 % per weight of cyclodextrin agent. Preferably, the pharmaceutical composition comprises 0.5 to 40 % per weight of cyclodextrin agent; more preferably 1.0 to 30 % per weight of cyclodextrin agent; and most preferably 1.0 to 16.0 % per weight of cyclodextrin agent with respect to the weight of the composition.

The composition of the invention may also comprise a disintegrant, a diluent, a processing aid, a lubricant, a gliding agent and a mixture therefore, as excipient.

The composition of the invention may comprise a disintegrant, as excipient.

The term "disintegrant" as used herein is defined as an accelerating agent of the disintegration of the tablet and the dispersion of the active ingredient in water or gastrointestinal fluids. The disintegrant may be present in the pharmaceutical composition in the form of a single compound or in the form of a mixture of compounds.

Examples of disintegrant are starches, pregelatinized starch, sodium croscarmellose, also referred to as cross-linked sodium carboxymethylcellulose, and cross-linked polyvinylpyrrolidone. Preferred disintegrants according to the present invention are cross-linked polyvinylpyrrolidone, sodium starch glycolate and sodium croscarmellose. More preferred disintegrant is sodium croscarmellose (crosslinked carboxymethylcellulose sodium).

Preferably, the compositions according to the present invention comprise 0.5 to 25 % per weight of disintegrant, more preferably 1.0 to 15 % per weight of disintegrant, most preferably 1.5 to 8 % per weight of disintegrant, with respect to the weight of the composition.

The composition of the invention may also comprise diluents as excipient.

The term "diluent" as used herein is defined as an agent used as filler in order to achieve the desired composition volume or weight. The diluent may be present in the pharmaceutical composition in the form of a single compound or in the form of a mixture of compounds.

Examples of diluent are, but not limited to, lactose, starch, pregelatinized starch, microcrystalline cellulose, silicified microcrystalline cellulose, cellulose acetate, dextrose, mannitol, sodium phosphate, potassium phosphate, calcium phosphate, fructose, maltose, sorbitol, or sucrose. Preferred diluents are lactose and starch. More preferably diluent is lactose monohydrate, anhydrous lactose or a mixture thereof. The best results have been obtained with a mixture of lactose monohydrate and anhydrous lactose.

Usually, the compositions according to the present invention comprise 5 to 95 % per weight of diluent with respect to the weight of the composition. Preferably, the compositions comprise 10 to 90 % per weight of diluent. More preferably, the compositions comprise 30 to 90% per weight of diluent with respect to the weight of the composition.

The composition of the invention may also comprise lubricant as excipient.

Examples of lubricants are, but not limited to, talc, magnesium stearate, calcium stearate, poloxamer, sodium lauryl sulfate, stearic acid, hydrogenated castor oil. Preferred lubricant according to the present invention is magnesium stearate.

Usually, the compositions according to the present invention comprise 0 to 5.50 % per weight of lubricant with respect to the total weight of the composition. Preferably, the compositions comprise 0.001 to 2.50 % per weight of lubricant. More preferably, the compositions comprise 0.01 to 2.0 % per weight of lubricant with respect to the total weight of the composition.

The pharmaceutical composition of the invention may also comprise a gliding agent as excipient.

Examples of gliding agents are, but not limited to colloidal silicon dioxide and talc. Preferred gliding agent according to the present invention is colloidal silicon dioxide.

Usually, the composition according to the present invention comprises 0 to 5.00 % per weight of gliding agent with respect to the total weight of the composition. Preferably, the composition comprises 0 to 2.50 % per weight of gliding agent. More preferably, the composition comprises 0 to 2.0 % per weight of gliding agent with respect to the total weight of the composition.

The compositions of the invention may also comprise other inactive ingredients such as a processing aid, a gliding agent and a mixture therefore, as excipient.

In one embodiment of the invention, the pharmaceutical composition comprises brivaracetam as active ingredient and 0.1% to 60% per weight of at least a cyclodextrin agent, with respect to the total weight of the composition.

Particularly, the pharmaceutical composition comprises
- Brivaracetam as active ingredient;
- 0.1 to 60 % per weight of at least a cyclodextrin agent;
- 0.5 to 25 % per weight of disintegrant; and
- 5 to 95 % per weight of diluent; with respect to the total weight of the composition.

Particularly, the pharmaceutical composition comprises
- Brivaracetam as active ingredient;
- 0.1 to 50% per weight of at least a cyclodextrin agent;
- 1.0 to 15 % per weight of disintegrant; and
- 10 to 90 % per weight of diluent; with respect to the total weight of the composition.

Particularly, the pharmaceutical composition comprises
- Brivaracetam as active ingredient;
- 1.0 to 30 % per weight of at least a cyclodextrin agent;
- 1.5 to 8 % per weight of disintegrant; and
- 30 to 90 % per weight of diluent; with respect to the total weight of the composition.

In another embodiment of the invention, the pharmaceutical composition comprises brivaracetam, as active ingredient; 0.1 to 60% per weight of at least a cyclodextrin agent with respect to the total weight of the composition; sodium croscarmellose; and lactose monohydrate.

Particularly, the pharmaceutical composition comprises
- Brivaracetam as active ingredient;
- 0.1 to 60 % per weight of at least a cyclodextrin agent;
- 0.5 to 25 % per weight of sodium croscarmellose; and
- 5 to 95 % per weight of lactose monohydrate; with respect to the total weight of the composition.

Particularly, the pharmaceutical composition comprises
- Brivaracetam as active ingredient;
- 0.1 to 50% per weight of at least a cyclodextrin agent;
- 2.0 to 15 % per weight of sodium croscarmellose; and
- 10 to 90 % per weight of lactose monohydrate; with respect to the total weight of the composition.

Particularly, the pharmaceutical composition comprises
- Brivaracetam as active ingredient;
- 1.0 to 30 % per weight of at least a cyclodextrin agent;
- 2.0 to 8 % per weight of sodium croscarmellose; and
- 30 to 90 % per weight of lactose monohydrate; with respect to the total weight of the composition.

In a preferred embodiment of the invention, the composition comprises 10.00 mg of brivaracetam, 2.70 mg of beta cyclodextrin, 19.40 mg of lactose monohydrate and 1.00 mg of sodium croscarmellose.

In a preferred embodiment of the invention, the composition comprises 10.00 mg of brivaracetam, 2.70 mg of beta cyclodextrin, 45.00 mg of lactose monohydrate and 2.00 mg of sodium croscarmellose.

In a preferred embodiment of the invention, the composition comprises 25.00 mg of brivaracetam, 6.75 mg of beta cyclodextrin, 48.50 mg of lactose monohydrate and 2.50 mg of sodium croscarmellose.

In a preferred embodiment of the invention, the composition comprises 50.00 mg of brivaracetam, 13.50 mg of beta cyclodextrin, 97.00 mg of lactose and 5.00 mg of sodium croscarmellose.

In a preferred embodiment of the invention, the composition is a tablet which comprises 10.00 mg of brivaracetam, 2.70 mg of beta cyclodextrin, 19.40 mg of lactose monohydrate and 2.00 mg of sodium croscarmellose; 19.30 mg of anhydrous lactose and 0.60 mg magnesium stearate.

In a preferred embodiment of the invention, the composition is a tablet which comprises 10.00 mg of brivaracetam, 2.70 mg of beta cyclodextrin, 45.00 mg of lactose monohydrate and 4.00 mg of sodium croscarmellose, 45.10 mg of anhydrous lactose and 1.20 mg magnesium stearate.

In a preferred embodiment of the invention, the composition is a tablet which comprises 25.00 mg of brivaracetam, 6.75 mg of beta cyclodextrin, 48.50 mg of lactose monohydrate and 5.00 mg of sodium croscarmellose, 48.25 mg of anhydrous lactose and 1.50 mg magnesium stearate.

In a preferred embodiment of the invention, the composition is a tablet which comprises 50.00 mg of brivaracetam, 13.50 mg of beta cyclodextrin, 97.00 mg of lactose monohydrate and 10.00 mg of sodium croscarmellose, 96.50 mg of anhydrous lactose and 3.00 mg magnesium stearate.

In a preferred embodiment of the invention, the composition is a tablet which comprises 9.2 mg of brivaracetam, 41.5 mg of lactose monohydrate, 2.5 mg of betacyclodextrin, 3.6 mg of sodium croscarmellose, 41.6 mg of lactose anhydrous, 1.1 mg of magnesium stearate and 0.5 mg of silicon dioxide colloidal.

The pharmaceutical composition of the invention is usually manufactured by direct compression.

The process for preparing compositions according to the invention comprises
- a first step wherein the active ingredient, and cyclodextrin agent, and all the excipients, are mixed; and
- a second step wherein the resulting blend is compressed and /or compacted in order to obtain tablets.

Possibly the tablets may be coated using a coating suspension or solution.

The compositions comprise the active ingredient, the diluent, the cyclodextrin agent, and the disintegrant and possibly the lubricant. The compositions are manufactured as follows: The active ingredient, the diluent, the cyclodextrin agent and the disintegrant are mixed using a planetary mixer, diffusion mixers, convection mixers and/or pneumatic mixer. Then possibly the lubricant is added. The blend is mixed. This blend is then compressed using a tabletting machine in order to obtain the tablets.

The main steps of the process for manufacturing tablets are as follows:
- Blending the cyclodextrin agent, croscarmellose sodium, lactose monohydrate, anhydrous lactose and the active ingredient: the blending operation can be achieved using diffusion mixers, convection mixers and/or pneumatic mixers.
- Compression of the blend: the tabletting operation can be performed using a tablet press.
- Film-coating: this operation can be performed using a pan coater or a gas suspension based coater and more preferably a perforated pan coater.

Cyclodextrin agent is used as a binder. No inclusion complexes are formed between the active ingredient and the cyclodextrin agent. The inventors have found a surprising binding effect of the cyclodextrin agent used in the pharmaceutical composition of the invention.

When the pharmaceutical composition of the invention is a tablet, the process may comprise a further film-coating step in which water, preferably purified water, is added to the film-coating agent and resulting suspension and/or solution is sprayed on the tablet.

In another aspect the present invention relates to a pharmaceutical composition comprising Brivaracetam useful for the treatment or prevention of a disease.

By the term "disease", we understand a disease selected from the group consisting of epileptogenesis, seizure disorders, convulsions, Parkinson's disease, dyskinesia induced by dopamine replacement therapy, tardive dyskinesia induced by administration of neuroleptic drugs, Huntington Chorea, and other neurological disorders including bipolar disorders, mania, depression, anxiety, attention deficit hyperactivity disorder (ADHD), migraine, cluster headache, trigeminal and other neuralgia, chronic pain, neuropathic pain, cerebral ischemia, cardiac arrhythmia, myotonia, cocaine and other substance abuse (e.g. alcohol, benzodiazepines, opiates, marijuana, barbiturates, amphetamines, other stimulants), stroke, myoclonus, dystonia,dyskinesia, tremor, essential tremor, simple or complex tics, Tourette syndrome, restless leg syndrome and other movement disorders, neonatal cerebral haemorrhage, amyotrophic lateral sclerosis, spasticity and degenerative diseases.

The term "treatment" as used herein, includes curative treatment and prophylactic treatment.

By "curative" is meant efficacy in treating a current symptomatic episode of a disorder or condition.

By "prophylactic" is meant prevention of the occurrence or recurrence of a disorder or condition.

The present invention concerns also a method for treatment of a human patient by using the pharmaceutical composition.

The present invention concerns also the pharmaceutical composition for use as a medicament for curing the said disease.

The present invention concerns also the use of the pharmaceutical composition for the manufacture of a medicament for a therapeutic application in the said disease.

Preferably said disease is selected from the group consisting essentially of epilepsy, Parkinson's disease, dyskinesia, migraine, tremor, essential tremor, bipolar disorders, chronic pain, neuropathic pain, or bronchial, asthmatic or allergic conditions. More preferably said disease is epilepsy.

The process used to prepare the pharmaceutical composition of the invention is easy, rapid, and cost efficient.

The amount of excipients was aimed to be as low as possible in order to keep a low tablet weight.

Another advantage of the pharmaceutical composition of the invention resides in the fact that proportional formulations are possible, so the same blend could be compressed as tablet cores of increasing size and mass depending on the dosage needed.

Adding cyclodextrin agent in the pharmaceutical composition of the invention results in compression ability and/or finally in-vitro dissolution results. Cyclodextrin agent does not require high compression pressures in order to produce tablets.

Brivaracetam is a very sticking compound (ability of adhere).The main advantage of cyclodextrin agent is to reduce the sticking of the blend during the preparation of the tablets, and specifically during the compression step.

Another advantage is to improve the drug dissolution.

The following examples are provided for illustrative purposes only and are not intended, nor should they be construed, as limiting the invention in any manner.

### Examples

### Example 1

A tablet (tablet A) is prepared by direct compression process with the following composition (table 1)

**Table 1 : composition 100 mg**

| Components | tablet A % |
|---|---|
| Brivaracetam | 37.0 |
| Lactose monohydrate | 48.4 |
| Beta cyclodextrin | 10.0 |
| Sodium croscarmellose | 3.8 |
| Magnesium stearate | 0.8 |

The brivaracetam as active ingredient, lactose monohydrate, the cyclodextrin agent and the sodium croscarmellose are mixed. Then magnesium stearate is added. Then the blend is compressed on a tablet machine to obtain the tablets.

**Table 2: results in % of 100 mg brivaracetam immediate release tablets (paddle method, 900 mL aqueous buffer, 50 rpm)**

| Time (hours) | % Brivaracetam dissolved in comparison with the total weight of brivaracetam in the composition |
|---|---|
| 0 | 0 |
| 0:15:00 | 100 |
| 0:30:00 | 100 |
| 0:45:00 | 100 |
| 1:00:00 | 100 |

Tablet 2 shows an immediate release of the Brivaracetam that complies with the *in vitro* dissolution requirements.

The in vitro dissolution profiles in water of tablets are determined according to the USP <711> (apparatus n° 2, 50 rpm, aqueous medium 900 mL, phosphate buffer pH 6). The dissolution was conducted at 37°C.

Adding cyclodextrin agent in the pharmaceutical composition of the invention results in compression ability. Cyclodextrin agent does not require high compression pressures in order to produce tablets.

The cyclodextrin agent is able to reduce sticking during compression and tabletting.

### Example 2

Tablets B, C and D are prepared by a direct compression process with the following composition (table 3). The process is identical to the process described in example 1.

**Table 3. Composition of tablets B, C and D**

| Amount (mg) | B | C | D |
|---|---|---|---|
| Brivaracetam | 10.00 | 25.00 | 50.00 |
| Beta cyclodextrin | 2.70 | 6.75 | 13.50 |
| Lactose monohydrate | 45.00 | 48.50 | 97.00 |
| Sodium croscarmellose | 4.00 | 5.00 | 10.00 |
| Anhydrous lactose | 45.10 | 48.25 | 96.50 |
| Magnesium stearate | 1.20 | 1.50 | 3.00 |
| Core tablet mass (mg) | 108 | 135 | 270 |

Tablets B, C and D show an immediate release of the Brivaracetam that complies with the *in vitro* dissolution requirements.

**Table 4: coated tablet C: results in % of 10 mg brivaracetam immediate release tablets (paddle method, 900 mL phosphate buffer pH 6.4, 50 rpm; performed on 6 tablets)**

| | | | | |
|---|---|---|---|---|
| Time (min) | 0 | 15 | 30 | 45 |
| mean (%) | 0 | 96 | 96 | 96 |

**Table 5: coated tablet D: results in % of 10 mg brivaracetam immediate release tablets (paddle method, 900 mL phosphate buffer pH 6.4, 50 rpm; performed on 6 tablets)**

| | | | | |
|---|---|---|---|---|
| Time (min) | 0 | 15 | 30 | 45 |
| mean (%) | 0 | 99 | 98 | 99 |

### Example 3

A tablet is prepared by a direct compression process with the following composition (table 6).

**Table 6: composition 50 mg**

| Components | tablet % |
|---|---|
| Brivaracetam | 18.5 |
| Lactose monohydrate | 35.9 |
| Beta cyclodextrin | 5.0 |
| Sodium croscarmellose | 3.7 |
| Magnesium stearate | 1.1 |
| Anhydrous lactose | 35.8 |

Test results show that the immediate release tablet complies with the *in vitro* dissolution requirements.

### Example 4

A tablet is prepared by dry compression process with the following composition (table 7).

**Table 7:**

| Components | Tablet (%) |
|---|---|
| Brivaracetam | 9.2 |
| Lactose monohydrate | 41.5 |
| Betacyclodextrin | 2.5 |
| Sodium Croscarmellose | 3.6 |
| Anhydrous lactose | 41.6 |
| Mg stearate | 1.1 |
| Silicon dioxide colloidal | 0.5 |

The main steps of the process for manufacturing tablets are as follows :
- Blending the cyclodextrin agent, croscarmellose sodium, lactose monohydrate, anhydrous lactose, silicon dioxide colloidal, Mg stearate and brivaracetam: the blending operation is achieved in order to have an homogenous blend.
- Compression: the tabletting operation can be performed using a tablet press;

The disintegration time for the above tablets is 1 minute 53 seconds when determined according to Eur. Ph. 2.9.1. Test results show that the immediate release tablet complies with the *in vitro* dissolution requirements.

### Example 5 (Reference example): All experiments were performed in accordance with the Guidelines of the local Ethical Committee for Animal Experimentation.

Epileptiform responses in hippocampal slices: Levetiracetam reduces epileptiform responses induced in rat hippocampal slices by high-K+/low-Ca2+ concentrations in the perfusion fluid and induced by bicuculline. The effect of brivaracetam on epileptiform responses induced by high-K+/low-Ca2+ concentrations or by bicuculline was examined in transverse hippocampal slices prepared from Sprague-Dawley rats according to previously reported standard procedures. The epileptiform responses were induced by passing from a normal perfusion of artificial cerebrospinal fluid (ACSF) (K+ 3 mM; Ca2+ 2.4 mM) to either high-K+/low-Ca2+ fluid (HKLCF) (K+ 7.5 mM; Ca2+ 0.5 mM) or to 5 M bicuculline methiodide (BMI)-containing ACSF.

Extracellular field potentials (FPs) were recorded in the CA3 area of the slices with 2 M NaCl-filled glass microelectrodes. The evoked FPs were recorded at 10-min intervals in response to fimbrial stimulation with constant current rectangular pulses that elicit a single population spike (PS) of 50-75% of the maximal amplitude when the slice is in ACSF. In the HKLCF model, 2 min of spontaneous activity were also recorded, in the middle of each 10-min interval between the recordings of evoked responses.

Either brivaracetam or levetiracetam was added to the bathing fluid of the slices 20 min before shifting from ACSF to either HKLCF or 5 M BMI-containing ACSF, and was kept in the perfusion fluid throughout the experiment.

Audiogenic seizures in mice: Genetically sound-sensitive male mice (16-28 g; n=10 per group), responding with wild running, clonic and tonic convulsions to an acoustic stimulation, were used. Audiogenic seizures were induced by an acoustic stimulus (90 dB, 10-20 kHz) applied for 30 s. The mice were pretreated with either saline, brivaracetam (i.p., 30 min) or levetiracetam (i.p., 60 min), and the proportion of mice protected against clonic convulsions was used as the end point to assess anticonvulsant activity.

Chemically induced seizures in mice:Pentylenetetrazol, 83 mg kg- 1 s.c., was used to evaluate the anticonvulsant properties of brivaracetam. The dose was selected based on dose-effect curves in saline-treated animals as the convulsive dose inducing clonic convulsions of all four extremities in 97% of the animals. Immediately after administration of the chemoconvulsant, the mice were placed individually in small plastic cages (25 13 8 cm) and observed for the presence of clonic convulsions in all four extremities, for 60 min. The occurrence of tonic convulsions (hindlimb extension) and mortality was also recorded during this interval. The proportion of mice protected against clonic convulsions was calculated and used as the end point for anticonvulsant activity.

### Results

Epileptiform responses in hippocampal slices: Changing the perfusion of rat hippocampal slices from the normal ACSF to HKLCF produced increasingly epileptiform FPs in the CA3 area in response to constant-current fimbrial stimulation. In control slices exposed to HKLCF alone, the PS1 amplitude progressively increased, reaching plateau values within 20 min (4.250.77 mV), nearly twofold higher than those recorded under ACSF perfusion (2.180.15 mV; means.d. for n=10 slices). Also, constant-current single stimuli-evoked bursts of repetitive PSs (that is, PS2, PS3 and so on) increased markedly in number in the first 30 min of HKLCF perfusion from the single PS1 to an average of 7.62.3 PS per evoked burst, and continued to increase slightly up to the end of the records, reaching an average of 8.81.6 PS per evoked burst after 80-min perfusion of HKLCF. Both brivaracetam and levetiracetam reduced these epileptiform responses. Upon 15-min perfusion of HKLCF, spontaneous field bursts occurred in 4 out of the 10 control slices exposed to HKLCF alone, whereas from 25 min in HKLCF to the end of the records, all control slices presented regular field bursting. Brivaracetam (3.2 M), but not levetiracetam (32 M), reduced the rate of this spontaneous bursting.

In vivo studies: In fully amygdala-kindled rats, brivaracetam induced a significant suppression in motor-seizure severity from a dose of 21.2 mg kg- 1, whereas levetiracetam induced a similar effect from a dose of 170 mg kg- 1. Brivaracetam also significantly reduced the after-discharge duration at the highest dose tested (212.3 mg kg- 1), whereas levetiracetam was inactive on this parameter up to 1700 mg kg- 1.

Audiogenic seizure-susceptible mice were protected against the expression of clonic convulsions by brivaracetam and levetiracetam. Brivaracetam, administered i.p. 30 min before seizure induction in mice, also protected against clonic convulsions induced by pentylenetetrazol and against tonic hindlimb extension induced by a maximal electroshock in mice, although with higher ED50 values.

Brivaracetam significantly suppressed spontaneous SWDs in GAERS rats from a dose of 2.1 mg kg- 1 with complete inhibition appearing at the highest dose tested (67.9 mg kg- 1).

Pretreatment with brivaracetam during corneal kindling of mice resulted in a significant reduction in the incidence of generalized motor seizures, and a similar incidence reduction was observed with levetiracetam at higher doses. Continued corneal stimulations following termination of treatment showed a persistent reduction in the incidence of generalized motor seizures in the group previously treated with the highest dose of brivaracetam.

## Claims

1. An oral pharmaceutical composition, in a solid tablet form, comprising an active ingredient and 0.1 % to 60% per weight of at least a cyclodextrin agent, as excipient, with respect to the total weight of the composition; the active ingredient being an 2-oxo-1-pyrrolidine derivative of formula (I), 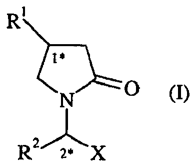 wherein,
R¹ is C₁₋₁₀ alkyl or C₂₋₆ alkenyl;
R² is C₁₋₁₀ alkyl or C₂₋₆ alkenyl;
X is -CONR⁴R⁵, -COOH, -COOR³ or -CN;
R³ is C₁₋₁₀ alkyl;
R⁴ is hydrogen or C₁₋₁₀ alkyl;
R⁵ is hydrogen or C₁₋₁₀ alkyl.

2. The composition according to claim 1, wherein R¹ is n-propyl or 2,2-difluorovinyl; R² is ethyl; and X is -CONH₂.

3. The composition according to claim 1 or 2, wherein the cyclodextrin agent is chosen among alpha cyclodextrin, beta cyclodextrin, hydroxypropyl beta cyclodextrin, methyl beta cyclodextrin, sulfobutyl beta cyclodextrin, gamma cyclodextrin, and hydroxypropyl gamma cyclodextrin.

4. The composition according to claim 3, wherein the cyclodextrin agent is a beta cyclodextrin.

5. The composition according to claim 1, 2, 3 or 4, wherein the cyclodextrin agent is a beta cyclodextrin having a water content between 5 and 16 % (w/w).

6. The composition according to any one of the claims 1 to 5, wherein the composition is a homogeneous blend of the active ingredient and all the excipients.

7. The composition according to any one of the claims 1 to 6, wherein the composition comprises 1.0 to 15.0 % per weight of cyclodextrin agent with respect to the weight of the composition.

8. The composition according to any one of the preceding claims, wherein the composition comprises a disintegrant, as excipient.

9. The composition according to claim 8, wherein the disintegrant is sodium croscarmellose.

10. The composition according to claim 8 or 9, wherein the composition comprises 1.5 to 8 % per weight of disintegrant, with respect to the weight of the composition.

11. The composition according to any one of the preceding claims, wherein the composition comprises a diluent, as excipient.

12. The composition according to claim 11, wherein the diluent is a mixture of lactose monohydrate and anhydrous lactose.

13. The composition according to claims 11 or 12, wherein the composition comprises 30 to 90% per weight of diluent with respect to the weight of the composition.

14. The composition according to any one of the preceding claims, wherein the composition comprises a lubricant, as excipient.

15. The composition according to claim 14, wherein the lubricant is magnesium stearate.

16. The composition according to claims 14 or 15, wherein the composition comprises 0.01 to 2.0 % per weight of lubricant with respect to the total weight of the composition.

17. The composition according to claim 1, wherein the composition comprises
- brivaracetam as active ingredient;
- 0.1 to 60 % per weight of at least a cyclodextrin agent;
- 0.5 to 25 % per weight of disintegrant; and
- 5 to 95 % per weight of diluent; with respect to the total weight of the composition.

18. A process for preparing a composition according to any of the preceding claims, wherein it includes at least a direct compression step.

19. A process according to claim 18, wherein it includes
- a first step wherein the active ingredient, and cyclodextrin agent, and all the excipients, are mixed; and
- a second step wherein the resulting blend is compressed in order to obtain tablets.

## Patentansprüche

1. Orale pharmazeutische Zusammensetzung in Form einer festen Tablette, umfassend einen Wirkstoff und 0,1 Gew.-% bis 60 Gew.-% mindestens eines Cyclodextrinmittels als Hilfsstoff, bezogen auf das Gesamtgewicht der Zusammensetzung, wobei der Wirkstoff ein 2-Oxo-1-pyrrolidin-Derivat der Formel (I) 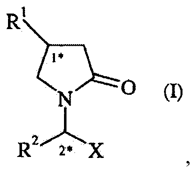 ist, wobei
R¹ C₁₋₁₀-Alkyl oder C₂₋₆-Alkenyl ist;
R² C₁₋₁₀-Alkyl oder C₂₋₆-Alkenyl ist;
X -CONR⁴R⁵, -COOH, -COOR³ oder -CN ist;
R³ C₁₋₁₀-Alkyl ist;
R⁴ Wasserstoff oder C₁₋₁₀-Alkyl ist;
R⁵ Wasserstoff oder C₁₋₁₀-Alkyl ist.

2. Zusammensetzung nach Anspruch 1, wobei R¹ n-Propyl oder 2,2-Difluorvinyl ist; R² Ethyl ist und X -CONH₂ ist.

3. Zusammensetzung nach Anspruch 1 oder 2, wobei das Cyclodextrinmittel ausgewählt ist aus Alphacyclodextrin, Betacyclodextrin, Hydroxypropylbetacyclodextrin, Methylbetacyclodextrin, Sulfobutylbetacyclodextrin, Gammacyclodextrin und Hydroxypropylgammacyclodextrin.

4. Zusammensetzung nach Anspruch 3, wobei das Cyclodextrinmittel ein Betacyclodextrin ist.

5. Zusammensetzung nach Anspruch 1, 2, 3 oder 4, wobei das Cyclodextrinmittel ein Betacyclodextrin mit einem Wassergehalt zwischen 5 und 16 Gew.-% ist.

6. Zusammensetzung nach einem der Ansprüche 1 bis 5, wobei die Zusammensetzung eine homogene Mischung aus dem Wirkstoff und allen Hilfsstoffen ist.

7. Zusammensetzung nach einem der Ansprüche 1 bis 6, wobei die Zusammensetzung 1,0 bis 15,0 Gew.-% Cyclodextrinmittel, bezogen auf das Gewicht der Zusammensetzung, umfasst.

8. Zusammensetzung nach einem der vorstehenden Ansprüche, wobei die Zusammensetzung einen Lösungsvermittler als Hilfsstoff umfasst.

9. Zusammensetzung nach Anspruch 8, wobei der Lösungsvermittler Natriumcroscarmellose ist.

10. Zusammensetzung nach Anspruch 8 oder 9, wobei die Zusammensetzung 1,5 bis 8 Gew.-% Lösungsvermittler, bezogen auf das Gewicht der Zusammensetzung, umfasst.

11. Zusammensetzung nach einem der vorstehenden Ansprüche, wobei die Zusammensetzung ein Verdünnungsmittel als Hilfsstoff umfasst.

12. Zusammensetzung nach Anspruch 11, wobei das Verdünnungsmittel ein Gemisch aus Lactosemonohydrat und wasserfreier Lactose ist.

13. Zusammensetzung nach Anspruch 11 oder 12, wobei die Zusammensetzung 30 bis 90 Gew.-% Verdünnungsmittel, bezogen auf das Gewicht der Zusammensetzung, umfasst.

14. Zusammensetzung nach einem der vorstehenden Ansprüche, wobei die Zusammensetzung ein Schmiermittel als Hilfsstoff umfasst.

15. Zusammensetzung nach Anspruch 14, wobei das Schmiermittel Magnesiumstearat ist.

16. Zusammensetzung nach Anspruch 14 oder 15, wobei die Zusammensetzung 0,01 bis 2,0 Gew.-% Schmiermittel, bezogen auf das Gesamtgewicht der Zusammensetzung, umfasst.

17. Zusammensetzung nach Anspruch 1, wobei die Zusammensetzung
- Brivaracetam als Wirkstoff;
- 0,1 bis 60 Gew.-% mindestens eines Cyclodextrinmittels;
- 0,5 bis 25 Gew.-% Lösungsvermittler und
- 5 bis 95 Gew.-% Verdünnungsmittel; bezogen auf das Gesamtgewicht der Zusammensetzung, umfasst.

18. Verfahren zur Herstellung einer Zusammensetzung nach einem der vorstehenden Ansprüche, wobei es zumindest einen direkten Kompressionsschritt umfasst.

19. Verfahren nach Anspruch 18, wobei es
- einen ersten Schritt, in dem der Wirkstoff und das Cyclodextrinmittel und die Hilfsstoffe gemischt werden; und
- einen zweiten Schritt, in dem das resultierende Gemisch unter Erhalt von Tabletten komprimiert wird, umfasst.

## Revendications

1. Composition pharmaceutique pour voie orale, sous la forme d'un comprimé solide, comprenant un principe actif et, comme excipient, de 0,1% à 60% en poids d'au moins un agent de cyclodextrine, par rapport au poids total de la composition ; le principe actif étant un dérivé de 2-oxo-1-pyrrolidine de formule (I), 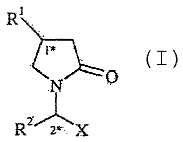 dans laquelle
R¹ est alcoyle ayant de 1 à 10 atomes de carbone ou alcényle ayant de 2 à 6 atomes de carbone ;
R² est alcoyle ayant de 1 à 10 atomes de carbone ou alcényle ayant de 2 à 6 atomes de carbone ;
X est CONR⁴R⁵, -COOH, -COOR³ ou -CN ;
R³ est alcoyle ayant de 1 à 10 atomes de carbone ;
R⁴ est hydrogène ou alcoyle ayant de 1 à 10 atomes de carbone ;
R⁵ est hydrogène ou alcoyle ayant de 1 à 10 atomes de carbone ;

2. Composition suivant la revendication 1, dans laquelle R¹ est n-propyle ou 2,2-difluorovinyle ; R² est éthyle et X est -CONH₂.

3. Composition suivant la revendication 1 ou 2, dans laquelle l'agent de cyclodextrine est choisi parmi une alpha cyclodextrine, une bêta cyclodextrine, une hydroxypropyle bêta cyclodextrine, une méthyle bêta cyclodextrine, une sulfobutyle bêta cyclodextrine, une gamma cyclodextrine et une hydroxypropyle gamma cyclodextrine.

4. Composition suivant la revendication 3, dans laquelle l'agent de cyclodextrine est une bêta cyclodextrine.

5. Composition suivant la revendication 1, 2, 3 ou 4, dans laquelle l'agent de cyclodextrine est une bêta cyclodextrine ayant une teneur en eau comprise entre 5 et 16% (poids/poids).

6. Composition suivant l'une quelconque des revendications 1 à 5, dans laquelle la composition est un mélange homogène du principe actif et de tous les excipients.

7. Composition suivant l'une quelconque des revendications 1 à 6, dans laquelle la composition comprend de 1,0 à 15,0% en poids d'agent de cyclodextrine par rapport au poids de la composition.

8. Composition suivant l'une quelconque des revendications précédentes, dans laquelle la composition comprend un agent de déliquescence comme excipient.

9. Composition suivant la revendication 8, dans laquelle l'agent de déliquescence est la croscarmellose de sodium.

10. Composition suivant la revendication 8 ou 9, dans laquelle la composition comprend de 1,5 à 8% en poids d'agent de déliquescence par rapport au poids de la composition.

11. Composition suivant l'une quelconque des revendications précédentes, dans laquelle la composition comprend un diluant comme excipient.

12. Composition suivant la revendication 11, dans laquelle le diluant est un agent de monohydrate de lactose et de lactose anhydre.

13. Composition suivant les revendications 11 ou 12, dans laquelle la composition comprend de 30 à 90% en poids de diluant par rapport au poids de la composition.

14. Composition suivant l'une quelconque des revendications précédentes, dans laquelle la composition comprend un lubrifiant comme excipient.

15. Composition suivant la revendication 14, dans laquelle le lubrifiant est le stéarate de magnésium.

16. Composition suivant les revendications 14 ou 15, dans laquelle la composition comprend de 0,01 à 2,0% en poids de lubrifiant par rapport au poids total de la composition.

17. Composition suivant la revendication 1, dans laquelle la composition comprend :
- du brivaracétame comme principe actif ;
- de 0,1 à 60% en poids d'au moins un agent de cyclodextrine ;
- de 0,5 à 25% en poids d'un agent de déliquescence ; et
- de 5 à 95% en poids d'un diluant par rapport au poids total de la composition.

18. Procédé de préparation d'une composition suivant l'une quelconque des revendications précédentes, qui comprend au moins un stade direct de compression.

19. Procédé suivant la revendication 18, qui comprend :
- un premier stade, dans lequel on mélange le principe actif et un agent de cyclodextrine et tous les excipients ; et
- un deuxième stade, dans lequel on comprime le mélange obtenu afin d'obtenir des comprimés.
